# EUROPEAN PATENT SPECIFICATION

(11) **EP 2 983 715 B1**
(45) Date of publication and mention of the grant of the patent: **29.05.2019**
(21) Application number: 14719066.4
(22) Date of filing: 10.04.2014
(51) Int. Cl.: A61K 47/14, A61K 9/10, A61K 9/50, A61K 31/4439

(54) **LONG-TERM OILY SUSPENSION WITH OMEPRAZOLE ENTERIC COATED BEADS**
LANGFRISTIGE ÖLIGE SUSPENSION MIT MAGENSAFTRESISTENTEN OMEPRAZOLKÜGELCHEN
SUSPENSION HUILEUSE LONGUE DURÉE CONTENANT DES PARTICULES D'OMÉPRAZOLE À ENROBAGE ENTÉRIQUE

(30) Priority: 12.04.2013 GB 201306720
(43) Date of publication of application: 17.02.2016
(73) Proprietor: Veriton Pharma Limited, Surrey KT13 0YF (GB)
(72) Inventor: MARCH, Graham, Weybridge Surrey KT13 0YF (GB); TITTERSHILL, Andrew, Weybridge Surrey KT13 0YF (GB); SOCORRO, Antonio, Weybridge Surrey KT13 0YF (GB)
(74) Representative: Hutter, Anton
(86) International application number: PCT/GB2014/051129
(87) International publication number: WO 2014/167342

(56) References cited:
- WO-A1-2006/026829
- WO-A2-2004/004682
- ES-A1- 2 283 172
- PILBRANT A ET AL: "DEVELOPMENT OF AN ORAL FORMULATION OF OMEPRAZOLE", SCANDINAVIAN JOURNAL OF GASTROENTEROLOGY, INFORMA HEALTHCARE, GB, vol. 20, 1 January 1985 (1985-01-01), pages 113-120, XP009044798, ISSN: 0036-5521, DOI: 10.3109/00365528509095824

## Description

The present invention relates to the claimed formulation. More particularly, it relates to a stable suspension formulation for an acid labile omeprazole, in particular, a stable formulation of omeprazole which is suitable for oral administration.

It should be noted that the scope of the invention is defined by the claims.

Any subject-matter falling outside the scope of the claims is provided for information purposes only.

Any references in the description to methods of treatment refer to the compounds, pharmaceutical compositions and medicaments of the present invention for use in a treatment of the human (or animal) body by therapy (or diagnosis).

Further a centipoise is one hundredth of a poise, or one millipascal-second (mPa·s) in Sl units (1 cP = 10-3 Pa·s = 1 mPa·s).

Omeprazole, Pantoprazole, Lansoprazole and other derivatives of benzimidazole are active proton pump inhibitors which decrease gastric secretion and as such are used in the treatment of, for example, dyspepsia, peptic ulcer disease, gastritus, gastroesophageal reflux disease, laryngopharyngeal reflux, gastric and duodenum ulceration and Zollinger-Ellison syndrome. These benzimidazoles are proton pump inhibitors that suppress gastric acid secretion by specific inhibition of the H+/K+-ATPase in the gastric parietal cell.

Although the present disclosure is applicable to a range of derivatives of benzimidazole, the invention will be given with reference to the claimed omeprazole.

Omeprazole, 5-methoxy-2((4-methoxy-3,5-dimethyl-2-pyridinyl)methylsulfinyl)-1H-benzimidazole, is disclosed in a number of patents and applications including EP 1830816 and EP1246622. Omeprazole is one of the most widely prescribed drugs. In some countries, it is also available "over the counter".

Generally omeprazole has been provided as tablets or capsules. Whilst, a formulation which can be administered orally may offer various advantages, finding a liquid formulation of omeprazole for oral administration has proved problematic as, on contact with acid, degradation occurs. Since the stomach is a highly acidic environment, if the active substance comes into contact with the stomach contents they degrade.

Specifically, omeprazole degrades with a half-life of less than 10 minutes in an environment with pH values below 4.0. At pH 6.5, the half life of omeprazole is 18 hours and at pH 11 about 300 days. It has therefore become commonplace to provide the omeprazole in a core together with alkaline constituents within an enteric coating.

It has therefore become desirable to seek a means of protecting the omeprazole both during storage and during the passage through the acidic environment of the stomach. It has therefore become commonplace to provide the omeprazole with an enteric coating to protect it from the acid.

Omeprazole is most commonly provided as enteric-coated granules located within capsules or as enteric-coated tablets. An enteric coating is a polymer coating barrier which is applied to the granules or capsules to protect the omeprazole from the acid in the stomach.

In some countries, omeprazole may be provided in injectable form. In this arrangement, it is generally provided in a combination pack comprising a vial of powder and a separate ampoule of a reconstituting solution.

Patients who struggle with swallowing tablets are advised to mix the granules from a capsule in a spoonful of, for example, apple sauce. The resulting mixture can then be swallowed. However it has to be taken immediately. These patients may also be provided with a powder for oral suspension which has to be mixed with water. However, the amount of water has to be carefully controlled and the mixture has to be allowed to thicken. The resultant thick suspension has to be taken within about 30 minutes and any excess cannot be stored for future use. Unfortunately, some people find this thick suspension unpleaseant to take. In addition, the requirements for extemporaneous production of the suspension can be difficult for the patient particularly if they are away from home. These difficulties may result in the patient not following the regimen.

A further problem with asking the patient to mix the granules with the apple sauce or the like is that the granules can become damaged if the patient is over vigorous in the mixing.

Whilst providing omeprazole as a suspension is desirable, and some suspensions are made to order in pharmacies or specialist manufacturing facilities, these have a very short shelf life. It is therefore not currently possible to batch manufacture a suspension with an acceptable shelf life.

It is therefore desirable to provide a liquid suspension which is stable such that the patient can readily take the required dosage as a liquid without having to mix powders and the like.

WO 2004/004682 describes a formulation which is suggested to have an extended shelf life in liquid form. This is suggested as being possible by seal-coating the omeprazole beads and by the use of an oily liquid. However, whilst the beads can be provided with the oily liquid, they are not in a true suspension. This may mean that in use, a volume of the suspension which may be taken by a patient on separate occasions may not contain the appropriate number of beads. It is also suggested that the formulation may be provided in single dose sachets. In this arrangement, the beads and liquids are added separately to the sachet.

According to the present invention there is provided a suspension comprising: microgranules having bead sizes in the range of 400 to 1200 microns of omeprazole; said omeprazole being suspended in a triglyceride ester of a fatty acid or acids; said suspension being stable for a least 1 month; wherein the viscosity of the suspension is from 1 centipoise to 5 000 centipoise, wherein the omeprazole is provided in the form of enteric coated beads, wherein the coating includes one or more layers selected from a disintegrant layer, a sealing layer and a control release layer, wherein the control release layer is formed from methacrylic acid or copolymers thereof, and the suspension additionally includes a preservative in an amount of 0,1 % (w/ v) to 0,5 % (w/v), and wherein the preservative is potassium sorbate.

The omeprazole will generally be provided in the form of enteric coated beads. In one arrangement, the omeprazole may be provided on a core. Any suitable biocompatible core may be used. A sugar core is conventional.

Whether or not the omeprazole is provided on a core, an enteric coating will generally be present. Any suitable enteric coating may be used. Examples of suitable enteric coating may be found in, for example, EP1830816, EP2456439, WO2004/004682, EP1246622, EP1030654, and EP1108425. However, enteric coatings provided by the seal coating method of WO2004/004682 are not generally preferred as it is believed that this means of applying the coating may lead to some ingress of water which can lead to hydrolysis and/or degradation of the omeprazole. The coating may be a single coating or a plurality of layers. Suitable layers include those providing a disintegrant, a sealing layer, and a control release layer. These may be provided in any suitable order.

Disintegrants are agents which promote the breakup of the pellet into smaller fragments. Suitable disintegrants include pregellatinized starch, microcrystalline cellulose, sodium bicarbonate, alginic acid, various ion exchange resins, modified starches such as sodium carboxymethyl starch, sodium starch glycolate, and modified cellulose such as sodium carboxymethyl cellulose with sodium starch glycolate being particularly preferred.

Suitable sealing layers include those formed from cellulose, such as hydroxypropyl methylcellulose, microcrystalline cellulose, polyvinylpyrrolidone, hydroxymethylcellulose, hydroxyethylcellulose, and dextran with hydroxypropyl methylcellulose being particularly preferred.

The control release layer is composed from methacrylic acid or copolymers thereof to form the layer.

The control layer may be pH and/or time-dependent. The layer is formed from methacrylic acid or copolymers thereof. A copolymer of methacrylic acid and ethyl acrylate is suitable.

Other components may be present in the enteric coating including emulsifiers, detackifiers, surfactants, flow aids, flavourants, and colourants.

The coating may be provided by any suitable method. The beads are within the range of 400 microns to 1200 microns. In one arrangement the beads may be within the range of from about 250 to about 600 microns. In another arrangement, the beads sizes are in the range of about 400 to about 600 microns. Methods of manufacture may result in some beads present being outside of this range however, the majority of the beads present will be within the range. Thus for example, in one example of where the beads are in the range of from about 400 microns to about 600 microns, not less than about 90% of the beads will pass through a 600 micron mesh and not less than about 90% of the beads are retained on a 250 micron mesh.

The beads may include from about 4% to about 15% of omeprazole with the remainder being made of the core and/or coating. In one arrangement, the beads may include from about 8.5% to about 10% of omeprazole.

Any suitable ester of a fatty acid or acids may be used in the suspension of the present invention. Triglyceride esters may offer certain advantages. Propylene glycol esters may be used. The ester may be formed with any suitable fatty acid or mixture of acids. The fatty acids may be derived from coconut or palm kernel oil. In one arrangement, a mixture of triglyceride esters of fatty acids may be used. The chain length of the fatty acid will be selected to provide the desired viscosity of the finished suspension. In one arrangement, the triglyceride ester component may be a mixture of triglyceride esters of C₈ to C₁₀ fatty acids. One example of a suitable triglyceride ester is that sold under the trade name Mygliol BP/USP. Myglycol 812 N may also be used.

The viscosity is from 1 centipoise to 5,000 centipoise or 2,000 centipoise.

The suspension may also include stabilisers. Any suitable stabiliser may be used. Suitable stabilisers include benzoic acid, calcium propoanate, potassium hydrogen sulphite, sodium nitrite, and silicon dioxide with silicon dioxide being particularly preferred. A combination of two or more stabilisers may be used.

Any suitable amount of stabiliser may be present. Generally the amount of stabiliser required will be of the order of from about 1% w/v to about 5% w/v and may be about 2% w/v to about 3% w/v.

The suspension includes preservatives. The preservative may have antimicrobial properties. The preservative used in the present suspension is potassium sorbate.

The amount of preservative required will be of the order of from 0.1% w/v to 0.5% w/v and may be about 0.2% w/v to about 0.3% w/v.

Sweeteners may be present. Suitable sweeteners include zylose, ribose, glucose, mannose, fructose, dextrose, sucrose, maltose, sorbitol, mannitol, glycerin, corn syrup, sodium glucamate, sodium saccharin, aspartame, and mixtures thereof with saccharin being particularly preferred. The amount of sweetener will be selected to give the desired level of sweetness. Generally the amount of sweetener, will be in the region of about 0.05% w/v to about 0.2% w/v.

Flavourings may also be included to make the suspension more palatable to the patient. Any suitable flavour may be used. Mint is a conventional flavouring but other flavourings such as fruit flavourings may be used. The amount of flavouring present in the suspension will be selected to achieve an acceptable flavouring.

The suspension may also include rafting agents which offer additional benefits in the treatment of the gastric acid problems. One suitable rafting agent is alginic acid. The rafting agent may be present in any suitable amount but may be present in from about 5% w/v to about 15% w/v and may be present in about 10% w/v.

The components of the present invention will generally be of sufficiently high purity and sufficiently low toxicity to render them suitable for administration to the patient.

The suspension is stable for at least 1 month, more preferably, at least 1 month, more preferably at least 2 months, more preferably at least 3 months, more preferably at least 4 months, more preferably at least 5 months, still more preferably at least 6 months. A product will generally be regarded as being no longer stable when an assay of the formulation shows that content of the active pharmaceutical ingredient has fallen below 90% of the stated label claim. i.e. if the formulation is 20mg of active in 5ml then assay shows that the active content has fallen below 18mg in 5ml. Stability can also be defined in terms of impurities and related substances. In the case of omeprazole it is 0.5% of the stated amount of the active.

The amount of omeprazole present in the solution will depend on the strength of final suspension required. Examples of suitable amounts will be 5 mg in 5 ml, 10 mg in 5ml and 20 mg in 5 ml, 30 mg in 5 ml, 40 mg in 5 ml, 50 mg in 5 ml, 60 mg in 5 ml, 70 mg in 5 ml and 80 mg in 5 ml.

The suspension of the present invention is particularly suitable as an active proton pump inhibitor, namely omeprazole, which decreases gastric secretion and as such may be used in the treatment of, for example, dyspepsia, peptic ulcer disease, gastritis, gastroesophageal reflux disease, laryngopharyngeal reflux, gastric and duodenum ulceration and Zollinger-Ellison syndrome. Thus the present invention also relates to the suspension of the first aspect of the present invention for use in the treatment of dyspepsia, peptic ulcer disease, gastritis, gastroesophageal reflux disease, laryngopharyngeal reflux, gastric and duodenum ulceration and Zollinger-Ellison syndrome. In addition, there is provided a method of treating dyspepsia, peptic ulcer disease, gastritis, gastroesophageal reflux disease, laryngopharyngeal reflux, gastric and duodenum ulceration and Zollinger-Ellison syndrome comprising administering to a patient in need thereof an effective amount of the suspension of the present invention.

The present invention also relates to packaged pharmaceutical formulations. Such packaged formulations include the suspension of the present invention in a suitable container and optionally containing instructions for using the composition to treat a patient suffering from dyspepsia, peptic ulcer disease, gastritus, gastroesophageal reflux disease, laryngopharyngeal reflux, gastric and duodenum ulceration and Zollinger-Ellison syndrome. In addition, there is provided a method of treating dyspepsia, peptic ulcer disease, gastritis, gastroesophageal reflux disease, laryngopharyngeal reflux, gastric and duodenum ulceration and Zollinger-Ellison syndrome. Frequency of disage will depend on the strength of the suspension, the particular disease or disorder being treated and the severity of the disease or disorder.

The suspension of the present invention may be administered alone or in combination with other medicaments.

Generally the patient will be a human but it may be a mammal, for example a domesticated companion animal, such as a dog or a cat or a livestock animal such as a cow, pig or horse.

The present invention will now be described with reference to the following examples:

### Example 1

Omeprazole 8.5% enteric coated micropellets were formed comprising a sugar sphere coated with omeprazole and having located thereon layers of sodium starch glycolate, hydroxypropyl methylcellulose and methacrylic acid.

### Example 2

One example of a suitable formulation is:

| Omeprazole BP | 5mg to 20mg (58.82 mg to 235.29mg of enteric coated beads from Example 1) |
|---|---|
| Alginic acid BP/USP | 10% w/v |
| Potassium sorbate BP | 0.2% w/v |
| Sweetener | 1% w/v |
| Silicon dioxide anhydrous BP | 2.5% w/v |
| Flavour | 0.02 % w/v |
| Titanium dioxide | 5% w/v |
| Potassium hydrogen carbonate BP | 2% w/v |
| Mygliol BP/USP | Make up to 5 ml volume |

This suspension is expected to have a stability of up to 6 months.

### Example 3

### A second example formulation is:

| Omeprazole | 5mg to 20mg (58.82 mg to 235.29mg of enteric coated beads from Example 1) |
|---|---|
| Potassium sorbate | 0.2% w/v |
| Saccharin | 0.1% w/v |
| Silicon dioxide | 3.0% w/v |
| Mint flavour | 0.1% |
| Mygliol 812N | Make up to 5ml |

This suspension is expected to have a stability of up to 6 months.

## Claims

1. A suspension comprising: microgranules having bead sizes in the range of 400 to 1200 microns of omeprazole suspended in a triglyceride ester of a fatty acid, wherein the viscosity of the suspension is from 1 to 5,000 mPa·s (or centipoise), wherein the omeprazole is provided in the form of enteric coated beads, wherein the coating includes one or more layers selected from a disintegrant layer, a sealing layer and a control release layer, wherein the control release layer is formed from methacrylic acid or copolymers thereof, and the suspension additionally includes a preservative in an amount of 0.1% (w/v) to 0.5% (w/v), and wherein the preservative is potassium sorbate.

2. A suspension according to claim 1, wherein the omeprazole is formed on a sugar core.

3. A suspension according to claim 1, wherein the chain length of the fatty acid is a mixture of C₈ to C₁₀ fatty acids.

4. A suspension according to claim 1, wherein the preservative is present in an amount of 0.2% w/v to 0.3% w/v.

5. A suspension according to any one of claims 1 to 4, additionally including one or more stabilisers, wherein the stabiliser is silicon dioxide.

6. A suspension according to claim 5, wherein the stabiliser is present in an amount of 1% w/v to 5% w/v or 2% w/v to 3% w/v.

7. A suspension according to any one of claims 1 to 6, additionally including a sweetener, wherein the sweetener is preferably present in an amount of 0.05% w/v to 0.2% w/v.

8. A suspension according to any one of claims 1 to 7, additionally including one or more rafting agents.

9. A suspension according to claim 8, wherein the rafting agent is alginic acid.

10. A suspension according to either claim 8 or claim 9, wherein the rafting agent is present in an amount of 5 to 15 % w/v, or 10 % w/v.

11. A suspension according to any one of claims 1 to 10, additionally including one or more excipients.

12. A suspension according to any one of claims 1 to 11, wherein the viscosity of the suspension is from 1,000 to 5,000 mPa·s (or centipoise) or is 2,000 mPa·s (or centipose).

13. A suspension according to any one of claims 1 to 12, wherein the amount of the omeprazole present is 5 mg in 5 ml, 10 mg in 5ml, 20 mg in 5 ml, 30 mg in 5 ml, 40 mg in 5 ml, 50 mg in 5 ml, 60 mg in 5 ml, 70 mg in 5 ml or 80 mg in 5 ml.

14. A suspension according to any one of claims 1 to 13, for use in the treatment of dyspepsia, peptic ulcer disease, gastritis, gastroesophageal reflux disease, laryngopharyngeal reflux, gastric and duodenum ulceration and Zollinger-Ellison syndrome.

## Patentansprüche

1. Suspension, Folgendes umfassend: Mikrogranulate aus Omeprazol mit Kügelchengrößen in dem Bereich von 400 bis 1200 Mikron, die in einem Triglyceridester einer Fettsäure suspendiert sind, wobei die Viskosität der Suspension von 1 bis 5.000 mPa·s (oder Zentipoise) beträgt,
wobei das Omeprazol in der Form von magensaftresistent beschichteten Kügelchen bereitgestellt ist, wobei die Beschichtung eine oder mehrere Schichten beinhaltet, die aus einer desintegrierbaren Schicht, einer abdichtenden Schicht und einer Kontrolltrennschicht ausgewählt sind, wobei die Kontrolltrennschicht aus Methacrylsäure oder Copolymeren davon gebildet ist und die Suspension zusätzlich ein Konservierungsmittel in einer Menge von 0,1 % (w/v) bis 0,5 % (w/v) beinhaltet und wobei das Konservierungsmittel Kaliumsorbat ist.

2. Suspension nach Anspruch 1, wobei das Omeprazol auf einem Zuckerkem gebildet ist.

3. Suspension nach Anspruch 1, wobei die Kettenlänge der Fettsäure eine Mischung aus C₈-bis C₁₀-Fettsäuren ist.

4. Suspension nach Anspruch 1, wobei das Konservierungsmittel in einer Menge von 0,2 % w/v bis 0,3 % w/v vorhanden ist.

5. Suspension nach einem der Ansprüche 1 bis 4, zusätzlich einen oder mehrere Stabilisatoren beinhaltend, wobei der Stabilisator Siliciumdioxid ist.

6. Suspension nach Anspruch 5, wobei der Stabilisator in einer Menge von 1% w/v bis 5 % w/v oder 2 % w/v bis 3 % w/v vorhanden ist.

7. Suspension nach einem der Ansprüche 1 bis 6, zusätzlich ein Süßungsmittel beinhaltend, wobei das Süßungsmittel vorzugsweise in einer Menge von 0,05 % w/v bis 0,2 % w/v vorhanden ist.

8. Suspension nach einem der Ansprüche 1 bis 7, zusätzlich ein oder mehrere Floßmittel beinhaltend.

9. Suspension nach Anspruch 8, wobei das Floßmittel Alginsäure ist.

10. Suspension nach Anspruch 8 oder Anspruch 9, wobei das Floßmittel in einer Menge von 5 bis 15 % w/v, oder 10 % w/v vorhanden ist.

11. Suspension nach einem der Ansprüche 1 bis 10, zusätzlich einen oder mehrere Hilfsstoffe beinhaltend.

12. Suspension nach einem der Ansprüche 1 bis 11, wobei die Viskosität der Suspension zwischen 1.000 bis 5.000 mPa·s (oder Zentipoise) beträgt oder 2.000 mPa·s (oder Zentipoise) beträgt.

13. Suspension nach einem der Ansprüche 1 bis 12, wobei die Menge des vorhandenen Omeprazols 5 mg in 5 ml, 10 mg in 5 ml, 20 mg in 5 ml, 30 mg in 5 ml, 40 mg in 5 ml, 50 mg in 5 ml, 60 mg in 5 ml, 70 mg in 5 ml oder 80 mg in 5 ml beträgt.

14. Suspension nach einem der Ansprüche 1 bis 13, zur Verwendung in der Behandlung von Dyspepsie, Magengeschwüren, Gastritis, gastroösophagealer Refluxkrankheit, laryngopharyngealem Reflux, gastrischer Ulzeration und Ulzeration des Doudenums und Zollinger-Ellison-Syndrom.

## Revendications

1. Suspension comprenant: des microgranules ayant des tailles de perle dans la plage de 400 à 1200 microns d'oméprazole suspendu dans un ester triglycéride d'un acide gras, dans laquelle la viscosité de la suspension est de 1 à 5000 mPa·S (ou centipoise), dans laquelle l'oméprazole est prévu sous la forme de perles à enrobage entérosoluble, dans laquelle l'enrobage inclut une ou plusieurs couches sélectionnées parmi une couche délitante, une couche d'étanchéité et une couche de régulation de libération, dans laquelle la couche de régulation de libération est formée d'acide méthacrylique ou de copolymères de celui-ci, et la suspension inclut en outre un conservateur dans une quantité de 0,1 % (m/v) à 0,5 % (m/v), et dans laquelle le conservateur est du sorbate de potassium.

2. Suspension selon la revendication 1, dans laquelle l'oméprazole est formé sur un coeur de sucre.

3. Suspension selon la revendication 1, dans laquelle la longueur de chaîne de l'acide gras est un mélange d'acides gras C₈ à C₁₀.

4. Suspension selon la revendication 1, dans laquelle le conservateur est présent dans une quantité de 0,2 % m/v à 0,3 % m/v.

5. Suspension selon l'une quelconque des revendications 1 à 4, incluant en outre un ou plusieurs stabilisants, dans laquelle le stabilisant est un dioxyde de silicium.

6. Suspension selon la revendication 5, dans laquelle le stabilisant est présent dans une quantité de 1% m/v à 5% m/v ou 2% m/v à 3% m/v.

7. Suspension selon l'une quelconque des revendications 1 à 6, incluant en outre un édulcorant, dans laquelle l'édulcorant est de préférence présent dans une quantité de 0,05% m/v à 0,2% m/v.

8. Suspension selon l'une quelconque des revendications 1 à 7, incluant en outre un ou plusieurs agents radeaux.

9. Suspension selon la revendication 8, dans laquelle l'agent radeau est un acide alginique.

10. Suspension selon l'une ou l'autre des revendications 8 ou 9, dans laquelle l'agent radeau est présent dans une quantité de 5 à 15% m/v, ou 10% m/v.

11. Suspension selon l'une quelconque des revendications 1 à 10, incluant en outre un ou plusieurs excipients.

12. Suspension selon l'une quelconque des revendications 1 à 11, dans laquelle la viscosité de la suspension est de 1000 à 5000 mPa·S (ou centipoise) ou est 2000mPa·S (ou centipose).

13. Suspension selon l'une quelconque des revendications 1 à 12, dans laquelle la quantité de l'oméprazole présent est 5 mg dans 5 ml, 10 mg dans 5 ml, 20 mg dans 5 ml, 30 mg dans 5 ml, 40 mg dans 5 ml, 50 mg dans 5 ml, 60 mg dans 5 ml, 70 mg dans 5 ml ou 80 mg dans 5 ml.

14. Suspension selon l'une quelconque des revendications 1 à 13, à utiliser dans le traitement d'une dyspepsie, une maladie d'ulcère gastriquye, une gastrite, une maladie de reflux gastro-oesophagien, un reflux laryngopharyngien, une ulcération gastrique et du duodenum et un syndrome de Zollinger-Ellison.
